# EUROPEAN PATENT APPLICATION

(11) **EP 1 870 910 A1**
(43) Date of publication of application: **26.12.2007**
(21) Application number: 06730011.1
(22) Date of filing: 20.03.2006
(51) Int. Cl.: H01B 19/00, C08L 89/00, H01B 3/00

(54) **DIELECTRIC BODY AND METHOD FOR PRODUCING SAME**

(30) Priority: 25.03.2005 JP 2005088222
(71) Applicant: NATIONAL INSTITUTE OF AGROBIOLOGICAL SCIENCES, Tsukuba-shi, Ibaraki 305-8602 (JP); Muroran Institute of Technology, National University Corporation, Muroran-shi, Hokkaido 050-8585 (JP)
(72) Inventor: TAMADA, Yasushi, 1-2, Owashi, Tsukuba-shi, Ibaraki, 3058 (JP); HIRAI, Shinji, o-cho, Muroran-shi, Hokkaido, 0508585 (JP)
(74) Representative: Vossius & Partner
(86) International application number: PCT/JP2006/306063
(87) International publication number: WO 2006/101223

(57) **Abstract**

This invention provides a method for producing a dielectric substance having excellent dielectric properties and being biodegradable, such method comprising subjecting silk protein to molding.

## Description

### Technical Field

The present invention relates to a method for producing a dielectric substance using silk protein, for example.

### Background Art

In general, the term "dielectric substance" refers to a substance that causes electric polarization but does not generate direct current (DC) electricity upon voltage application. This term is synonymous with the term "electric insulator."

A dielectric polymer comprising an organic polymer has excellent molding processability. Accordingly, such dielectric polymers are alternately laminated with electrode layers to form a film capacitor, and the resulting film capacitor is mainly used for a CR time constant circuit, bypass, coupling, resonant circuit, or filter. In accordance with the recent advancement of information technology, dielectric substances are considered to be important materials for printed circuit boards of computers, cellular phones, and the like.

In recent years, shortening of propagation wavelengths on boards has been demanded for high-frequency circuit boards for the purpose of reducing the size of circuits. In general, a signal propagation wavelength becomes smaller as a relative dielectric constant of a board material becomes greater. Accordingly, use of a material with a high relative dielectric constant is required for a high-frequency circuit board. As the radiowave frequency becomes greater, the activity of thermal conversion in the circuit of the printed circuit board becomes greater, which results in a greater transmission loss. This requires the use of a material with a smaller dielectric loss tangent for a high-frequency circuit board.

Speeding-up of information processing requires speeding-up of signal propagation speed. To this end, the size of a semiconductor device may be reduced or the wiring of the device may be shortened via high-density packaging. In addition, use of a material with a low relative dielectric constant may be critical for a printed circuit board, in order to speed up the signal propagation speed, for example.

In order to develop a dielectric substance that can be used as a material for such high-frequency circuit board or capacitor, use of various polymer materials, such as polypropylene, polyethylene terephthalate, or polyethylene naphthalate, has been examined. Such polymer materials have a relative dielectric constant of 3 and a dielectric loss tangent of 0.01 or smaller at 1 kHz and thus are excellent as dielectric substances in low frequency regions. In high frequency regions exceeding 1 MHz, for example, the dielectric loss tangents of such polymer materials are significantly increased, and they considerably exceed 0.1 at 10 MHz. Thus, use of such polymer materials in high frequency regions is disadvantageously limited.

Rapid advancements in home appliances or information technology devices shorten the turnover duration of the devices used for such appliances or devices. This requires disposal of large quantities of film capacitors and printed circuit boards. In the future, such disposal could pose a serious environmental concern.

In order to avoid such problem, use of biodegradable materials is required for film capacitors or printed circuit boards. At present, polylactate is known as a biodegradable polymer material. The dielectric properties of polylactate, however, are substantially equivalent to those of the aforementioned polymer materials. Thus, it is difficult to use polylactate as a material for a dielectric substance of a high-frequency circuit board or capacitor.

### Disclosure of the Invention

Under the above circumstances, the present invention provides a method for producing a dielectric substance having excellent dielectric properties and being biodegradable.

The present inventors have conducted concentrated studies in order to attain the above object. As a result, they discovered that a dielectric substance having excellent dielectric properties and being biodegradable could be produced with the use of silk protein as a starting material. This has led to the completion of the present invention.

The present invention includes the following.
(1) A method for producing a dielectric substance comprising molding silk protein.
(2) The method for producing a dielectric substance according to (1), wherein the silk protein is fibroin.
(3) The method for producing a dielectric substance according to (1), wherein the molding comprises a step of pressurization.
(4) The method for producing a dielectric substance according to (3), wherein the step of pressurization is carried out at 300°C or lower.
(5) A dielectric substance comprising, as a constituent, silk protein.
(6) The dielectric substance according to (5), wherein the silk protein is fibroin.
(7) The dielectric substance according to (5), wherein the relative dielectric constant is between 0.5 and 30 at 0.1 kHz to 300 GHz.
(8) The dielectric substance according to (5), wherein the dielectric loss is between 0.001% and 5% at 0.1 kHz to 300 GHz.

This description includes part or all of the contents as disclosed in the description and/or drawings of Japanese Patent Application No. 2005-088222, which is a priority document of the present application.

### Brief Description of the Drawings

Fig. 1 is a photograph showing a mold product produced in Example 1.
Fig. 2 shows the results of measuring dielectric properties (i.e., the relative dielectric constant and the dielectric loss) of the mold product produced in Example 1.
Fig. 3 shows the results of measuring dielectric properties (i.e., the relative dielectric constant and the dielectric loss) of the mold product produced in Example 2.

### Best Modes for Carrying out the Invention

Hereafter, the present invention is described in detail.

According to the method for producing a dielectric substance of the present invention, a dielectric substance with excellent dielectric properties can be produced from silk protein. The term "dielectric substance" used herein refers to a substance that causes electric polarization but does not generate direct current (DC) electricity upon voltage application.

In the method for producing a dielectric substance of the present invention, any silk protein produced from silkworms such as domesticated silkworms, wild silkworms, or wild silkmoths (*Antheraea yamamai*) may be used. Silk is composed of two types of silk proteins, i.e., fibroin and sericin. Fibroin constitutes a fiber. Sericin constitutes a silk thread, which is a gelatinous substance that covers the outer surface of the fiber constituted by silk fibroin. According to the present invention, use of fibroin as silk protein is preferable from the viewpoint of productivity.

Silk protein can be extracted and purified from a cocoon generated by a silkworm. Silk protein can also be extracted from the silk gland of a silkworm. Particularly preferably, silk protein is prepared from the cocoon of a domesticated silkworm, from the viewpoint of ease of production process. For example, silk protein extract can be extracted from a cocoon or silk gland with the use of an extraction solvent. The aforementioned silk protein extract can be concentrated. Further, the silk protein extract can be subjected to filtration, centrifugation, dialysis, purification, or other means to remove insoluble matter, the extraction solvent, or the like from the extract. In the present invention, the term "silk protein extract" refers to various extracts obtained with the use of a solvent, such as an aqueous silk protein solution obtained by the above extraction technique, a diluted solution thereof, or a concentrate thereof, for example.

In the method for producing a dielectric substance according to the present invention, a fiber, powder, film, sponge, gel, or solution containing silk protein prepared in accordance with various known techniques, for example, can also be used, in addition to the aforementioned silk protein extract.

In the present invention, silk protein may be chemically modified via chemical processing or graft polymerization, and the resulting modified silk protein can be used. Further, modified silk protein produced by a transgenic silkworm or the like can also be used. Furthermore, a chemically or enzymatically degraded silk protein can also be used. Also, a mixture of such modified silk proteins or a mixture of silk protein produced by various silkworms can also be used.

In the method for producing a dielectric substance of the present invention, various aqueous solvents or water-soluble polymers may be added to silk protein, and the resultants can be used. Examples of aqueous solvents that can be added to silk protein include water and glycerol. Examples of water-soluble polymers include polyvinyl pyrrolidone, polyvinyl alcohol, and polyhydroxy methacrylate. The amounts of aqueous solvents or water-soluble polymers added to silk protein are preferably 0% to 50%, and particularly preferably 0% to 40%, by weight of silk protein. When the amounts exceeds 50%, the stability or the dielectric constant of the produced dielectric substance may deteriorate.

In the method for producing a dielectric substance of the present invention, silk protein is subjected to molding to form a mold product. In the present invention, such mold product is a dielectric substance. For example, molding is carried out via film formation by casting and drying of silk protein (e.g., a silk protein extract or silk protein solution), film formation by coating a board with silk protein, or pressurization. Pressurization can be carried out by, for example, pulse energization sintering or hot pressing. The shape of a mold product is not particularly limited, and it can be in the form of a film, plate, round bar, disc, square bar, or rod, for example.

In the case of film formation via casting and drying, the concentration of silk protein is not particularly limited. For example, an extract or solution containing about 0.05% to 20% silk protein may be cast on the surface of a plastic petri dish or the like, followed by drying. Thus, a mold product can be obtained. Drying can be preferably carried out at a temperature range between, for example, 4°C and 300°C, although the temperature is not particularly limited thereto. Alternatively, post-treatment, such as pressurization or processing with an organic solvent, may be carried out following film formation. Pressurization can be preferably carried out at a pressure range between, for example, 0.1 MPa and 100 MPa, although the pressure is not particularly limited thereto.

In the case of film formation via coating of a board, the concentration of silk protein is not particularly limited. For example, a plastic, metal, or ceramic board can be coated with an extract or solution containing about 0.05% to 20% silk protein. Thus, a mold product can be obtained. After coating, drying or pressurization may be carried out. The drying or pressurization can be carried out at any temperature or pressure without particular limitation; however, drying is preferably carried out at 4°C to 300°C and pressurization is preferably carried out at 0.1 MPa to 100 MPa.

In the case of molding via pressurization, for example, silk protein is introduced into a cell of a desired configuration, and pressure is applied for a given period of time. If the temperature is over 300°C, carbonization of the mold product may disadvantageously advance. Accordingly, pressurization is carried out at 300°C or lower, preferably 250°C or lower, and particularly preferably 200°C or lower. On the contrary, a homogeneous mold product may not be obtained if pressurization is carried out at a temperature below room temperature (e.g., 23°C). Accordingly, pressurization is effectively carried out at room temperature or higher, preferably 60°C or higher, and particularly preferably 80°C or higher. Also, pressurization is carried out at 5 MPa or higher. If the pressure is lower than 5 MPa, it may be impossible to obtain a homogeneous mold product. If the pressure is over 100 MPa, the mold product may become fragile. Thus, pressurization is effectively carried out at 5 MPa to 100 MPa, and preferably 10 MPa to 50 MPa, for example. When heating is carried out in addition to pressurization, these procedures can be carried out simultaneously or successively and separately. The duration of pressurization can be adequately determined in accordance with the duration required for preparing a mold product. It is preferably between 10 minutes and 120 minutes. If the duration of pressurization is less than 10 minutes, it may be impossible to obtain a homogeneous mold product. If the duration of pressurization is over 120 minutes, the mold product may become fragile. Subsequently, the mold product obtained via pressurization is removed from the cell and then cooled. Thus, a mold product having a desired configuration (i.e., a dielectric substance) can be obtained.

The thus-obtained mold product can be used in that state. Alternatively, the mold product can be subjected to mechanical processing to form a desired configuration.

According to the method for producing a dielectric substance of the present invention described above, a dielectric substance with excellent dielectric properties can be obtained. Examples of dielectric properties include the relative dielectric constant and the dielectric loss.

The term "relative dielectric constant" refers to a ratio (ε/ε₀) of the dielectric constant ε of a substance to the dielectric constant *in vacuo* ε₀(= 8.854 X 10⁻¹²).

The dielectric constant of the dielectric substance of the present invention is measured by using, for example, an LCR meter or a network analyzer in high frequency regions. When the relative dielectric constant determined based on the dielectric constant measured by the above method is 0.5 to 30, preferably 2 to 10, and particularly preferably 3 to 7 in the frequency range between 0.1 kHz and 300 GHz, and particularly in high frequency regions between 1 MHz and 300 GHz, for example, the relative dielectric constant of the dielectric substance of the present invention can be determined to be sufficient.

The term "dielectric loss" refers to the amount of the energy lost as heat when an alternate electric field is applied to the dielectric substance.

The dielectric loss of the dielectric substance of the present invention is measured by using, for example, an LCR meter or a network analyzer in high frequency regions. When the dielectric loss measured by the above method is 0.001% to 5%, preferably 0.001% to 3%, and particularly preferably 0.01% to 3% in the frequency range between 0.1 kHz and 300 GHz, and particularly in high frequency regions between 1 MHz and 300 GHz, for example, the dielectric loss of the dielectric substance of the present invention can be determined to be sufficient.

According to the method for producing a dielectric substance of the present invention, a dielectric substance having excellent dielectric properties comprising, as a constituent, silk protein can be obtained. The resulting dielectric substance comprises, as a constituent, silk protein, and it is biodegradable. Thus, environmental burdens resulting from the production and disposal of such dielectric substance are reduced. Further, the dielectric substance of the present invention does not increase the dielectric loss in high frequency regions and it has excellent mechanical properties. Accordingly, the dielectric substance of the present invention can be easily processed into various shapes and can be extensively used for film capacitors, printed circuit boards, and the like.

In order to lower the relative dielectric constant and the dielectric loss tangent, the dielectric substance of the present invention is used as a base material, and air having a relative dielectric constant of 1 is introduced to make the dielectric substance porous. Alternatively, addition of fillers made of thermosetting resin, glass fiber, or powder having a low dielectric constant and low dielectric loss tangent may be effective. The term "dielectric loss tangent" refers to the tangent (tan δ) of the phase difference angle and the complementary angle, δ, (dielectric-loss angle) of a current component having the same frequency as the applied voltage of the currents that flow in the dielectric substance when a sinusoidal voltage is applied to the dielectric substance.

The dielectric substance may be made porous by regulating the conditions for preparing the dielectric substance or thin film of the present invention, by adding a foaming agent, or by adding silk fibers or silk powders with excellent crystallinity.

When fillers are added, various thermosetting resins including PTFE each having a relative dielectric constant of 2.1 and a dielectric loss tangent of 0.0002 at 10 GHz, D glass having a relative dielectric constant of 4.2 and a dielectric loss tangent of 0.0024 at 2.54 GHz, Q glass having a relative dielectric constant and a dielectric loss tangent smaller than the above mentioned values, and the like can be effectively used as fillers.

Hereafter, the present invention is described in greater detail with reference to the following examples, although the technical scope of the present invention is not limited thereto.

### [Example 1] Production of the dielectric substance of the present invention and dielectric properties thereof

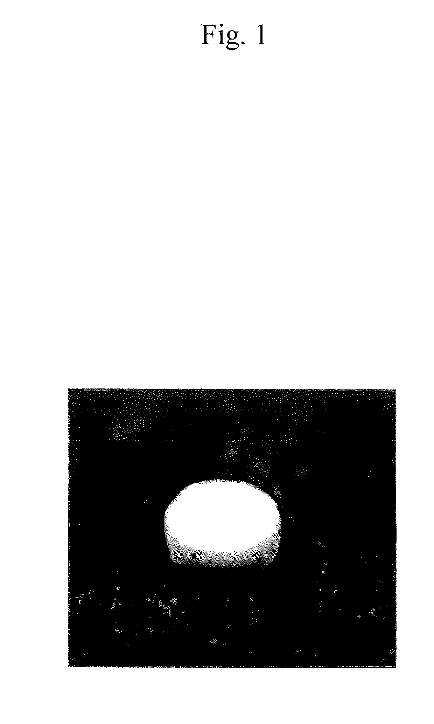

Distilled water was added to 1 g of silk powder (type IM, Kanebo, Ltd.) in an amount such that it accounted for 20% by mass of the resultant. Subsequently, heating and pressurization were carried out using an apparatus for pulse energization sintering *in vacuo* at 4.0 Pa or lower to produce a mold product (i.e., a dielectric substance). Molding was carried out at 20 MPa and 200°C (a rate of temperature increase: 20 K/min) for a retention time of 0 minutes and at a rate of cooling of 30 K/min. Fig. 1 shows a photograph of the resulting mold product.

The Vickers hardness, the Young's modulus, and water absorption of the resulting mold product were measured.

The Vickers hardness was measured by a test method in accordance with JIS Z 2244 (the Vickers hardness test-test method).

The Young's modulus was measured by a test method in accordance with JIS R 1602 (a testing method for elastic moduli of fine ceramics).

Water absorption was determined by soaking the mold product in water at 37°C for 24 hours, determining the mass increase resulting from soaking, and dividing the determined value by the mass of the sample before soaking.

The resulting Vickers hardness, the Young's modulus, and water absorption of the mold product were found to be 28.5, 52.9 Pa, and 22.6%, respectively.

Further, dielectric properties (the relative dielectric constant and the dielectric loss) of the resulting mold product were measured. Dielectric properties were measured using an LCR meter or a network analyzer in high frequency regions.

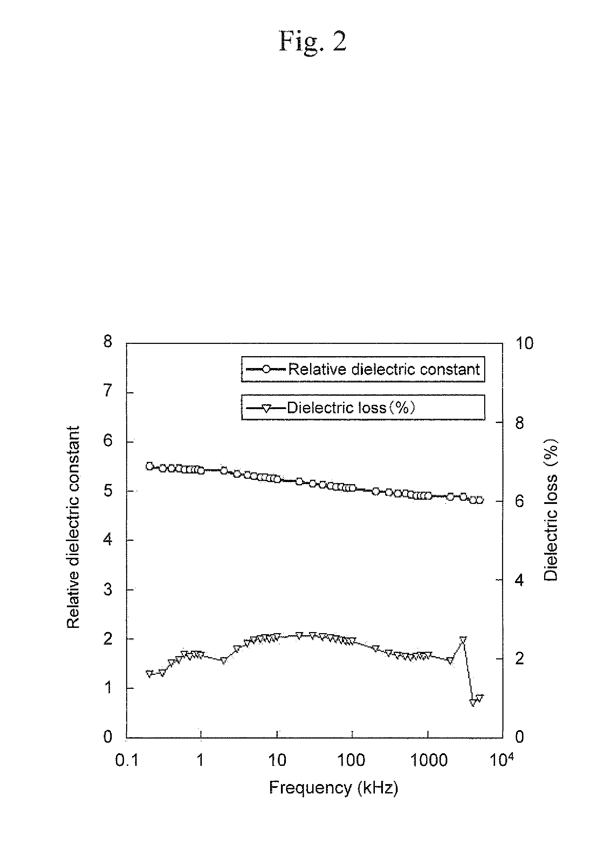

Fig. 2 shows the results of measuring the relative dielectric constant and the dielectric loss of the mold product. In Fig. 2, a line plotted with outlined circles represents a relative dielectric constant relative to the frequency, and a line plotted with outlined triangles represents a dielectric loss relative to the frequency.

As is apparent from Fig. 2, the mold product obtained in the present Example exhibited a stable relative dielectric constant over a wide frequency range.

### [Example 2] Production of the dielectric substance of the present invention using a silk protein film and dielectric properties thereof

A cocoon of a domesticated silkworm (10 g) was added to 1 liter of an aqueous solution of 0.5% sodium carbonate, and the resultant was refined by boiling for 1 hour. The resulting silk protein (silk fibroin, 3 g) was dissolved in 50 ml of a 9 M LiBr solution, and the resulting solution was dialyzed against pure water to prepare a silk protein solution. The silk protein concentration in the resulting silk protein solution was adjusted to 2%. Subsequently, the silk protein solution was cast on a polystyrene petri dish and treated at 50°C for 12 hours to prepare a silk protein film. Further, several dozen silk protein films were laminated, distilled water was added thereto such that it accounted for 30% by mass of the resultant, and the resultant was heated and pressurized using an apparatus for pulse energization sintering *in vacuo* of 4.0 Pa or lower to prepare a mold product (i.e., a dielectric substance). Molding was carried out at 20 MPa and 200°C (a rate of temperature increase: 20 K/min) for a retention time of 0 minutes and at a rate of cooling of 30 K/min.

After molding, the resulting mold product was dried at 373 K for 24 hours. Following drying, dielectric properties (the relative dielectric constant and the dielectric loss) of the mold product were measured in the same manner as in the case of Example 1.

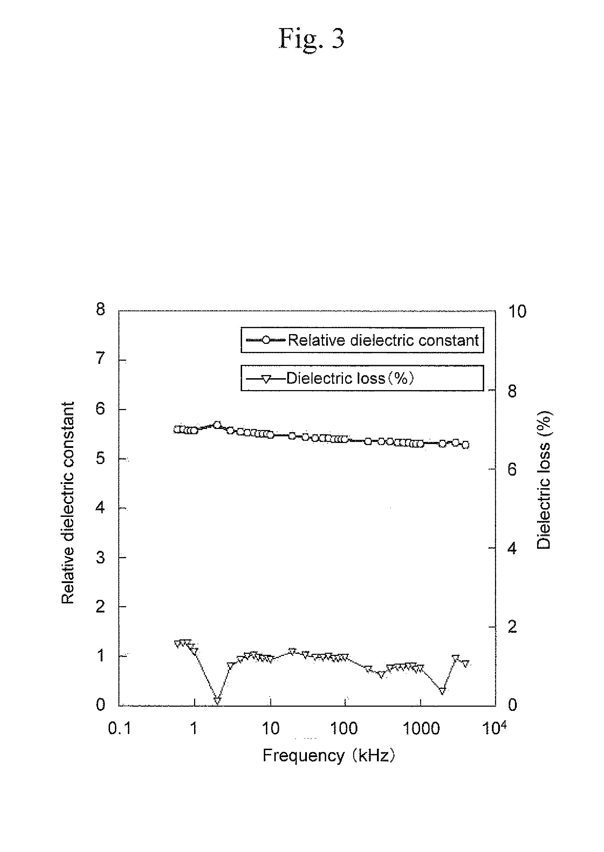

Fig. 3 shows the results of measuring the relative dielectric constant and the dielectric loss of the mold product. In Fig. 3, a line plotted with outlined circles represents a relative dielectric constant relative to the frequency, and a line plotted with outlined triangles represents a dielectric loss relative to the frequency.

As is apparent from Fig. 3, the mold product obtained in the present Example exhibited a stable relative dielectric constant and a small dielectric loss over a wide frequency range.

Dielectric properties of the dielectric substance obtained in the present Example were measured in a frequency range up to 300 GHz. As a result, the relative dielectric constant was found to be 5.8 at 150 Hz, it declined as the frequency increased, and it declined to 4 at 300 GHz. Further, the dielectric loss tangent (tan δ) of the dielectric substance obtained in the present Example gradually increased from 0.01 at 150 Hz along with the frequency, and it was found that the dielectric loss tangent would not exceed 0.08 at 300 GHz.

### [Comparative Example] Dielectric properties of conventional polymer materials

As a comparative example, dielectric properties (the relative dielectric constant and the dielectric loss) of conventional polymer materials (i.e., polyethylene terephthalate, polyphenylene sulfide, and polyethylene naphthalate) were measured. The relative dielectric constant and the dielectric loss were measured in the same manner as in the case of Example 1. The results are shown in Table 1. Table 1 shows the relative dielectric constant relative to the frequency of 1 kHz and the dielectric loss (%) relative to the frequencies of 1 kHz, 1 MHz, and 10 MHz. For the purpose of comparison, dielectric properties of the mold product obtained in Example 2 are also shown in Table 1.

**Table 1**

| | Relative dielectric constant (1 kHz) | Dielectric loss (%) (1 kHz) | Dielectric loss (%) (1 MHz) | Dielectric loss (%) (10 MHz) |
|---|---|---|---|---|
| Polyethylene terephthalate (PET) | 3.1 | 0.5 | 2.2 | >10 |
| Polyphenylene sulfide (PPS) | 3 | 0.05 | 0.3 | >10 |
| Polyethylene naphthalate (PEN) | 3 | 0.8 | 1.2 | >10 |
| Mold product obtained in Example 2 | 5.5 | 1.5 | 1 | 1 |

As is apparent from Table 1, conventional polymer materials exhibit excellent relative dielectric constant and dielectric loss in low-frequency regions. In high-frequency regions, however, significant increase was observed in dielectric loss, and, particularly, the dielectric loss exceeded 10% in high-frequency regions of 10 MHz or higher. Accordingly, use of conventional polymer materials is difficult in high-frequency regions. As is apparent from Table 1, the mold product obtained in Example 2 exhibits a small dielectric loss of about 1% in high-frequency regions of 10 MHz. Thus, the dielectric substance of the present invention has dielectric properties satisfactory for use in high-frequency regions.

### Industrial Applicability

According to the method for producing a dielectric substance of the present invention, a dielectric substance with excellent dielectric properties that are useful in various fields can be effectively produced. Also, the dielectric substance of the present invention is produced from silk protein and thus is biodegradable.

All publications, patents, and patent applications cited herein are incorporated herein by reference in their entirety.

## Claims

1. A method for producing a dielectric substance comprising molding silk protein.

2. The method for producing a dielectric substance according to claim 1, wherein the silk protein is fibroin.

3. The method for producing a dielectric substance according to claim 1, wherein the molding comprises a step of pressurization.

4. The method for producing a dielectric substance according to claim 3, wherein the step of pressurization is carried out at 300°C or lower.

5. A dielectric substance comprising, as a constituent, silk protein.

6. The dielectric substance according to claim 5, wherein the silk protein is fibroin.

7. The dielectric substance according to claim 5, wherein the relative dielectric constant is between 0.5 and 30 at 0.1 kHz to 300 GHz.

8. The dielectric substance according to claim 5, wherein the dielectric loss is between 0.001% and 5% at 0.1 kHz to 300 GHz.
